Europäisches Patentamt

(19) European Patent Office

Office européen des brevets

(11) EP 0 591 443 B1

# (12) FASCICULE DE BREVET EUROPEEN

(45) Date de publication et mention
de la délivrance du brevet:
**23.10.1996 Bulletin 1996/43**

(21) Numéro de dépôt: **92915137.1**

(22) Date de dépôt: **26.06.1992**

(51) Int. Cl.$^6$: **A61K 7/48**, A61K 7/06

(86) Numéro de dépôt international:
**PCT/FR92/00597**

(87) Numéro de publication internationale:
**WO 93/00067 (07.01.1993 Gazette 1993/02)**

(54) **UTILISATION DES COMPOSITIONS COSMETIQUES CONTENANT DES OLIGOSACCHARIDES**

VERWENDUNG VON KOSMETISCHEN ZUSAMMENSETZUNGEN ENTHALTEND
OLIGOSACCHARIDEN

USE OF COSMETIC COMPOSITIONS CONTAINING OLIGOSACCHARIDES

(84) Etats contractants désignés:
**BE CH DE DK ES FR GB IT LI NL SE**

(30) Priorité: **27.06.1991 FR 9107955**

(43) Date de publication de la demande:
**13.04.1994 Bulletin 1994/15**

(73) Titulaire: **BIOEUROPE**
**F-31400 Toulouse (FR)**

(72) Inventeurs:
• **LAMOTHE, Jean-Pierre, Henri, Georges**
**F-31000 Toulouse (FR)**
• **MARCHENAY, Yves, Gilbert**
**F-92100 Boulogne (FR)**
• **MONSAN, Pierre, Frédéric**
**F-31700 Blagnac (FR)**
• **PAUL, François, Marie, Bernard**
**F-31400 Toulouse (FR)**

• **PELENC, Vincent**
**6 bis, rue Bernard-Mule**
**F-31400 Toulouse (FR)**

(74) Mandataire: **Colas, Jean-Pierre**
**Cabinet de Boisse**
**37, avenue Franklin D. Roosevelt**
**75008 Paris (FR)**

(56) Documents cités:
EP-A- 0 480 640     FR-A- 2 231 323
FR-A- 2 515 486     GB-A- 2 186 066
US-A- 4 364 837

• PATENT ABSTRACTS OF JAPAN vol. 11, no. 167
(C-425)
• DATABASE WPIL Week 8549, Derwent
Publications Ltd., London, GB; AN 85-305539
• DATABASE WPIL Week 9133, Derwent
Publications Ltd., London, GB; AN 91-241116
• PATENT ABSTRACTS OF JAPAN vol. 13, no. 224
(C-599)

**Description**

L'invention concerne l'utilisation de certains oligosaccharides pour la fabrication d'une composition visant à favoriser le développement d'une microflore cutanée ou vaginale bénéfique.

L'homme et les animaux sont l'hôte de populations de microorganismes qui se développent naturellement tant à la surface que dans les cavités de leur corps. Ces flores microbiennes, ou microflores endogènes, sont caractéristiques de l'espèce et de la région du corps où elles se développent. En contact étroit avec leur hôte, certains microorganismes sont connus comme ayant une action bénéfique sur la peau ou la muqueuse vaginale, par exemple en maintenant un environnement légèrement acide et/ou en participant à la protection de l'organisme vis-à-vis d'infections par des microorganismes pathogènes peu ou pas présents habituellement sur la peau ou sur la muqueuse vaginale.

Il serait donc utile de disposer de cosmétiques créant un milieu favorable au développement de la flore endogène bénéfique et orientant le métabolisme de l'ensemble de la microflore endogène de façon à ce que cette dernière participe au maintien du bon équilibre physico-chimique de la peau et des muqueuses tout en ne favorisant pas concomitamment le développement de microorganismes pathogènes.

Au cours de recherches approfondies, la demanderesse a trouvé que certains oligosaccharides constituaient des produits facilement métabolisables par plusieurs souches bénéfiques de la microflore cutanée et de la flore vaginale. Ainsi on a observé, au cours d'études de culture in vitro, une métabolisation importante des glucooligosaccharides par des souches non pathogènes telles que Micrococcus kristinae, Micrococcus sedentarius, Staphylococcus capitis, Corynebacterium xerosis et Lactobacillus pentosus. Par contre, des souches pathogènes ou opportunistes telles que Staphylococcus aureus, Gardnerella vaginalis et Propionibacterium acnes ne métabolisent pas, ou très faiblement, ces oligosaccharides.

Par ailleurs, on a observé également que, en présence de ces oligosaccharides, certaines souches telles que Lactobacillus pentosus, Micrococcus kristinae, Gardnerella vaginalis, Propionibacterium avidum et Propionibacterium granulosum, acidifient le milieu de culture. Les Lactobacillus produisent en particulier de l'acide lactique.

L'invention concerne donc l'utilisation d'un composé choisi dans le groupe constitué par les glucooligosaccharides, les fructooligosaccharides, les $\alpha$- et $\beta$-galacto-oligosaccharides et les mélanges de ceux-ci qui sont facilement métabolisables par des souches bénéfiques de la microflore cutanée ou vaginale, mais peu ou pas métabolisables par des souches pathogènes ou opportunistes, pour la fabrication d'une composition pour le traitement de la peau ou de la muqueuse vaginale visant à favoriser le développement d'une microflore cutanée ou vaginale bénéfique.

L'invention concerne aussi l'utilisation d'un compose choisi dans le groupe constitué par les glucooligosaccharides, les fructooligosaccharides, les $\alpha$- et $\beta$-galacto-oligosaccharides et les mélanges de ceux-ci qui sont facilement métabolisables par des souches bénéfiques de la microflore cutanée ou vaginale, mais peu ou pas métabolisables par des souches pathogènes ou opportunistes, pour la fabrication d'un agent favorisant la croissance de la microflore cutanée ou vaginale bénéfique.

L'invention concerne, en outre, l'utilisation cosmétique d'un produit favorisant la croissance de la microflore cutanée bénéfique comprenant un composé choisi dans le groupe constitué par les glucooligosaccharides, les fructooligosaccharides, les $\alpha$- et $\beta$-galacto-saccharides et les mélanges de ceux-ci qui sont facilement métabolisables par des souches bénéfiques de la microflore cutanée, mais peu ou pas métabolisables par des souches pathogènes ou opportunistes.

Des glucosaccharides utilisables dans l'invention sont notamment les glucooligosaccharides répondant à la formule générale :

$$(O\text{-}\alpha\text{-}D\text{-glucopyranosyl})_n\text{-}A$$

où A est le résidu d'un sucre accepteur de glucose choisi parmi le maltose, l'isomaltose, l'isomaltotriose, l'$\alpha$-glucoside de méthyle et le glucose, et $\underline{n}$ = 1 à 10, les liaisons glucosidiques étant de type $\alpha(1{\rightarrow}6)$ et, facultativement, $\alpha(1{\rightarrow}2)$ et/ou $\alpha(1{\rightarrow}3)$, la liaison $\alpha(1{\rightarrow}2)$, si présente, étant située à l'extrémité non réductrice ou constituant un point de ramification.

Les oligosaccharides ci-dessus peuvent être produits par des procédés connus de synthèse enzymatique à l'aide d'enzyme glucosyl-transferase provenant de bactéries Leuconostoc mesenteroides, en présence de saccharose et d'un sucre accepteur de glucose choisi parmi le maltose, l'isomaltose, l'isomaltotriose, l'$\alpha$-glucoside de méthyle et le glucose.

De tels procédés sont décrits dans la littérature, par exemple dans EP-A-0 325 872, par Paul et coll. dans Carbohydrate Research, 149 (1986), pages 433-441, et dans US-A-2 726 190. Des souches de Leuconostoc mesenteroides préférées pour la mise en oeuvre du procédé de synthèse sont les souches NRRL B-512(F) (pour la production de glucooligosaccharides à liaisons $\alpha(1{\rightarrow}6)$ seulement) et NRRL B-1299 (pour la production de glucooligosaccharides à liaisons $\alpha(1{\rightarrow}6)$ et $\alpha(1{\rightarrow}2)$).

Comme sucre accepteur de glucose, on préfère tout particulièrement le maltose.

Des fructo-oligosaccharides utiles, isolément ou en mélange, sont notamment ceux répondant à la formule générale GFn ou à la formule générale Fm où G est un résidu de glucose, F un résidu de fructose, n vaut de 1 à 10, en particulier 2 à 4, et m vaut de 1 à 10, en particulier 2 à 4. De tels fructo-oligosaccharides existent à l'état naturel dans un certain nombre de végétaux tels que oignon, ail, artichaut, chicorée, etc... On peut les synthétiser par action sur le saccharose d'un enzyme de transfert, la fructosyl transférase, extraite d'un Aspergillus. On peut également préparer des fructo-oligosaccharides ramifiés utiles à partir de l'inuline de racines de chicorée par des processus enzymatiques, comme décrit dans WO-A1-91 13076.

Des fructo-oligosaccharides (appelés aussi oligofructoses) utiles sont disponibles dans le commerce sous les désignations Actilight[®] (vendu par Beghin-Meiji Industries, Paris, France ou Raftilose[®] (produit par la Raffinerie Tirlemontoise, Tienen, Belgique).

Des $\alpha$-galactooligosaccharides utiles, isolément ou en mélange, sont notamment ceux qui possèdent un résidu saccharose à une de leurs extrémités et répondent à la formule générale :

$$(\alpha\text{-Gal}(1 \longrightarrow 6))_n \underbrace{\alpha\text{-Glu}(1 \longrightarrow 2)\beta\text{-fructose}}_{\text{saccharose}},$$

où n vaut de 1 à 3, à savoir le raffinose (n = 1), le stachyose (n = 2) et le verbascose (n = 3) qui peuvent être extraits du soja. Des $\alpha$-galactooligosaccharides utiles sont disponibles dans le commerce auprès de la Société japonaise Calpis Food Industries. Voir également JP-A2-3 151 854 qui décrit ces composés. Des $\beta$-galactooligosaccharides utiles, isolément ou en mélange, sont notamment ceux qui possèdent un résidu lactose à une de leurs extrémités et répondent à la formule :

$$(\beta - \text{Gal}(1 \longrightarrow a))_n \underbrace{\beta - \text{Gal}(1 \longrightarrow 4)\,\text{glucose}}_{\text{lactose}}$$

où n = 1, 2 ou 3 et a = 2, 3, 4 et 6, l'isomère $\beta$ (1 → 6) étant majoritaire. De tels $\beta$-galactooligosaccharides peuvent être obtenus par synthèse enzymatique, par exemple par action de la $\beta$-galactosidase en présence de lactose concentré, et sont disponibles dans le commerce auprès de la Société japonaise Yakult Honsha. Voir également JP-A2-2 156 893 et EP-A-272 095 qui décrivent ces composés et leur préparation.

Grâce à l'utilisation de l'oligosaccharide, on peut maintenir un milieu favorable au développement de la flore endogène bénéfique.

La quantité d'oligosaccharide à incorporer dans la composition ou agent produit selon l'invention peut aller de 0,1 % en poids à 20 % et plus, de préférence de 1 à 10 % en poids. En dessous de 0,1 % en poids, l'effet de l'oligosaccharide devient négligeable. Par ailleurs, il n'y a pas d'avantages particuliers à incorporer plus de 20 % en poids de l'oligosaccharide, bien que cela soit parfaitement possible. Des considérations d'ordre économique amènent aussi à limiter la quantité d'oligosaccharide à 20 % en poids, de préférence à 10 % en poids, et très avantageusement à 5 % en poids.

Habituellement, le constituant oligosaccharide sera constitué d'un mélange d'oligosaccharides dans la mesure où les procédés de fabrication de ces composés produisent de tels mélanges.

Outre le constituant oligosaccharide, les compositions ou agents produits selon l'invention peuvent contenir les ingrédients habituellement incorporés dans ce genre de compositions. Il va de soi évidemment qu'il convient d'éviter d'incorporer aux compositions des ingrédients dont les propriétés interféreraient avec le but recherché qui est de favoriser le développement de la microflore, cutanée ou vaginale, bénéfique et le maintien de conditions acides.

Ainsi, il convient d'éviter d'incorporer des ingrédients bactéricides en des proportions qui annihileraient la microflore endogène ou des ingrédients conférant à la composition un caractère basique prononcé.

Par exemple, il sera bon d'éviter d'utiliser de fortes proportions d'agents tensio-actifs ioniques, tels que le laurylsulfate de sodium, dont les propriétés bactéricides sont bien connues. Si on a besoin d'incorporer une forte proportion d'un agent tensio-actif à la composition (par exemple dans le cas d'un savon liquide ou d'un shampooing) on utilisera plutôt un agent tensio-actif non-ionique, tel qu'un alkyl glucoside ou un ester de dialkyle.

Cependant, incorporer aux compositions produites selon l'invention une faible proportion (par exemple moins de 1 % en poids) d'agent conservateur ayant une action bactériostatique et, éventuellement, anti-fongique peut être tolérée pour permettre la conservation desdites compositions pendant de longues périodes.

Avantageusement, les compositions ou agents produits selon l'invention contiennent un tampon acide ajustant le pH de la composition dans la gamme de 5 à 7, de préférence de 5 à 6, tel qu'un tampon acide malique/malate de sodium, de façon à favoriser la réimplantation rapide de la microflore.

Les oligosaccharides peuvent être incorporés à des compositions très diverses, telles que savon liquide, shampooing, lait corporel, crème pour le visage, gel vaginal, etc...

Ces compositions peuvent être préparées facilement par les techniques couramment en usage dans l'industrie des cosmétiques.

Afin d'illustrer l'invention, on donne ci-après les exemples non-limitatifs suivants de compositions produites selon l'invention.

Dans les exemples 1 à 5, le constituant oligosaccharide utilisé était un mélange de glucooligosaccharides produits par la société BIOEUROPE conformément aux enseignements de EP-A-0 325 872 en utilisant du maltose comme sucre accepteur de glucose et de la glucosyltransferase provenant de la souche Leuconostoc mesenteroides NRLL B-1299. Ce mélange de glucooligosaccharides à liaisons $\alpha(1\rightarrow6)$ et $\alpha(1\rightarrow2)$ était formé majoritairement d'oligosaccharides des degrés de polymérisation 3 à 7 (n = 1 à 5).

Dans les exemples toutes les proportions indiquées sont en % en poids.

Exemple 1

| Savon liquide | |
|---|---|
| Glucooligosaccharides "Bioeurope" | 5 |
| Agent tensio-actif non-ionique (ORAMIX®NS10) | 18 |
| Epaississant (Sepigel® 305) | 2 |
| Conservateur (Sepicide®HB) | 0,7 |
| Tampon Acide malique/malate de sodium (qsp pH 5,5) # | 0,10 |
| Eau | qsp 100 |
| Notes : ORAMIX®NS10 est un alkyl glucoside vendu par la société SEPPIC. | |

SEPIGEL®305 est un agent épaississant et stabilisant pour émulsions, vendu par la société SEPPIC, se présentant sous forme d'une émulsion fluide, neutre. Il s'agit d'un matériau polymère référencé sous l'appellation: polyacrylamide (et) isoparaffine (et) laureth-7 (nom CTFA).

SEPICIDE®HB est un agent conservateur liquide, vendu par la société SEPPIC, pour la protection des produits d'hygiène et de beauté contre la contamination microbienne. Il s'agit d'esters de méthyle, éthyle, propyle et butyle d'acide parahydroxybenzoique en association avec de l'alcool phénoxyéthylique.

Exemple 2

| Shampooing | |
|---|---|
| Glucooligosaccharide "Bioeurope" | 5 |
| Agent tensio-actif de l'Ex. 1 | 10 |
| Polysorbate 80 | 5 |
| Conservateur de l'Ex. 1 | 0,7 |
| Tampon de l'Ex. 1 | (qsp pH 5,5) # 0,1 |
| Eau | qsp 100 |
| Note : Polysorbate 80 est un émulsifiant. Il s'agit d'un mono-oleate de polyoxyéthylène(20)-sorbitane. | |

## Exemple 3

| Lait corporel (émulsion) | |
|---|---|
| Glucooligosaccharide "Bioeurope" | 5 |
| Octanoate de cétyle et de stéaryle (phase grasse) | 10 |
| Polysorbate 80 | 3,6 |
| Palmitate de glycol (phase grasse) | 2,0 |
| Epaississant de l'Ex. 1 | 1,5 |
| Ester de sorbitane (agent émulsifiant) | 0,7 |
| Conservateur de l'Ex. 1 | 0,7 |
| Tampon de l'Ex. 1 | qsp pH 6 |
| Eau | qsp 100 |

## Exemple 4

| Crème pour le visage (émulsion) | |
|---|---|
| Glucooligosaccharide "Bioeurope" | 5 |
| Octanoate de cétyle et de stéaryle (phase grasse) | 3 |
| Polysorbate 80 | 5 |
| Micropearl M 100 (agent nacrant vendu par la société japonaise Matsumoto) | 3 |
| Conservateur de l'Ex. 1 | 0,7 |
| Tampon de l'Ex. 1 | qsp pH 6 |
| Eau | qsp 100 |

## Exemple 5

| Gel vaginal | |
|---|---|
| Glucooligosaccharide "Bioeurope" | 5 |
| Epaississant de l'Ex. 1 | 3 |
| Conservateur de l'Ex. 1 | 0,7 |
| Tampon de l'Ex. 1 | qsp pH 6 |
| Eau | qsp 100 |

## Exemple 6

Des compositions semblables à celles des Exemples 1 à 5 ont été préparées si ce n'est qu'on a remplacé le glucooligosaccharide "Bioeurope" sus-décrit par un glucooligo-saccharide produit de façon semblable au glucooligosaccharide "Bioeurope" mais en employant de la glucosyl-transferase provenant de la souche Leuconostoc mesenteroides NRLL B-512(F).

Le glucooligosaccharide obtenu comportait seulement des liaisons glucosidiques $\alpha(1 \rightarrow 6)$.

Les compositions ainsi produites avaient des propriétés comparables à celles des Exemples 1-5.

Exemple 7

Des compositions semblables à celles des Exemples 1 à 5 ont été préparées si ce n'est qu'on a remplacé le gluco-oligosaccharide "Bioeurope" sus-décrit par des quantités identiques des fructo-oligosaccharides Actilight® P disponibles auprès de la Société Beghin-Meiji Industries.

Les compositions ainsi produites avaient des propriétés comparables à celles des Exemples 1-5.

Exemple 8

Des compositions semblables à celles des Exemples 1 à 5 ont été préparées si ce n'est qu'on a remplacé le gluco-oligosaccharide "Bioeurope" sus-décrit par des quantités supérieures de 25% du sirop (à 80% d'extrait sec) de fructo-oligosaccharides Raftilose® L55.

Exemple 9

Des compositions semblables à celles des Exemples 1 à 5 ont été préparées si ce n'est qu'on a remplacé le gluco-oligosaccharide "Bioeurope" sus-décrit par des quantités similaires des α-galactooligosaccharides extraits du soja disponibles auprès de la Société Calpis Food Industry Co., Ltd.

Exemple 10

Des compositions semblables à celles des Exemples 1 à 5 ont été préparées si ce n'est qu'on a remplacé le gluco-oligosaccharide "Bioeurope" sus-décrit par des quantités similaires des β-galactooligosaccharides disponibles auprès de la Société japonaise Yakult Honsha.

Les compositions ainsi produites avaient des propriétés comparables à celles des Exemples 1-5.

**Revendications**

1.  Utilisation d'un composé choisi dans le groupe constitué par les glucooligosaccharides, les fructooligosaccharides, les α- et β-galacto-oligosaccharides et les mélanges de ceux-ci qui sont facilement métabolisables par des souches bénéfiques de la microflore cutanée ou vaginale, mais peu ou pas métabolisables par des souches pathogènes ou opportunistes, pour la fabrication d'une composition pour le traitement de la peau ou de la muqueuse vaginale visant à favoriser le développement d'une microflore cutanée ou vaginale bénéfique.

2.  Utilisation d'un composé choisi dans le groupe constitué par les glucooligosaccharides, les fructooligosaccharides, les α- et β-galacto-oligosaccharides et les mélanges de ceux-ci qui sont facilement métabolisables par des souches bénéfiques de la microflore cutanée ou vaginale, mais peu ou pas métabolisables par des souches pathogènes ou opportunistes, pour la fabrication d'un agent favorisant la croissance de la microflore cutanée ou vaginale bénéfique.

3.  Utilisation cosmétique d'un produit favorisant la croissance de la microflore cutanée bénéfique comprenant un composé choisi dans le groupe constitué par les glucooligosaccharides, les fructo-oligosaccharides, les α-et β-galacto-oligosaccharides et les mélanges de ceux-ci qui sont facilement métabolisables par des souches bénéfiques de la microflore cutanée, mais peu ou pas métabolisables par des souches pathogènes ou opportunistes.

4.  Utilisation selon la revendication 1, caractérisée en ce que la composition contient 0,1 à 20% en poids dudit oligosaccharide.

5.  Utilisation selon la revendication 1, 2 ou 3, caractérisée en ce que l'oligosaccharide est un gluco-oligosaccharide de la formule générale :

$$(O\text{-}\alpha\text{-}D\text{-glucopyranosyl})_n\text{-}A$$

où A est le résidu d'un sucre accepteur de glucose choisi parmi le maltose, l'isomaltose, l'isomaltotriose, l'α-glucoside de méthyle et le glucose, et $\underline{n}$ = 1 à 10, les liaisons glucosidiques étant de type $\alpha(1\rightarrow6)$ et, facultativement, $\alpha(1\rightarrow2)$ et/ou $\alpha(1\rightarrow3)$, la liaison $\alpha(1\rightarrow2)$, si présente, étant située à l'extrémité non réductrice ou constituant un point de ramification.

**6.** Utilisation selon la revendication 1, 2 ou 3, caractérisée en ce que l'oligosaccharide est un fructo-oligosaccharide de l'une des formules générales

$$GFn \text{ ou } Fm$$

où G est un résidu de glucose, F un résidu de fructose, n = 1 à 10 et m = 1 à 10.

**7.** Utilisation selon la revendication 1, 2 ou 3 caractérisée en ce que l'oligosaccharide est un $\alpha$-galactooligosaccharide ou un mélange d'$\alpha$-galactooligosaccharides répondant à la formule générale :

$$(\alpha\text{-Gal}(1 \to 6))_n \, \alpha\text{-Glu}(1 \to 2)\beta\text{-fructose}$$

, où n = 1, 2 ou 3.

**8.** Utilisation selon la revendication 1, 2 ou 3 caractérisée en ce que l'oligosaccharide comprend un mélange de $\beta$-galacto-oligosaccharides de la formule générale

$$(\beta\text{-Gal}(1 \to a))_n \, \beta\text{-Gal}(1 \to 4) \text{ glucose}$$

où n = 1, 2 ou 3 et a = 2, 3, 4 ou 6, l'isomère $\beta(1 \to 6)$ étant majoritaire.

**9.** Utilisation selon la revendication 1, caractérisée en ce que la composition comprend, en outre, un agent tensio-actif non-ionique.

**10.** Utilisation selon la revendication 1, caractérisée en ce que la composition comprend, en outre, un agent tampon ajustant le pH dans la gamme de 5 à 7.

**11.** Utilisation selon l'une quelconque des revendications 1 à 10, caractérisée en ce que la composition est sous la forme d'un savon liquide, d'un shampooing, d'un lait corporel ou d'une crème pour le visage.

**12.** Utilisation selon l'une quelconque des revendications 1, 2 et 4 à 10, caractérisée en ce que la composition est sous la forme d'un gel vaginal.

## Claims

**1.** Use of a compound selected from the group consisting of gluco-oligosaccharides, fructo-oligosaccharides, $\alpha$- and $\beta$-galacto-oligosaccharides and mixtures thereof, which can be easily metabolized by beneficial strains of the skin or vaginal microflora, but which are scarcely or not capable of being metabolized by pathogenic or opportunistic strains, for the preparation of a composition for the treatment of the skin or of the vaginal mucous membrane aiming to favor the development of a beneficial skin or vaginal microflora.

**2.** Use of a compound selected from the group consisting of gluco-oligosaccharides, fructo-oligosaccharides, $\alpha$- and $\beta$-galacto-oligosaccharides and mixtures thereof, which can be easily metabolized by beneficial strains of the skin or vaginal microflora, but which are scarcely or not capable of being metabolized by pathogenic or opportunistic strains, for the preparation of an agent which favors the growth of beneficial skin or vaginal microflora.

**3.** Cosmetic use of a product which favors the growth of beneficial skin or vaginal microflora, comprising a compound selected from the group consisting of gluco-oligosaccharides, fructo-oligosaccharides, $\alpha$- and $\beta$-galacto-oligosaccharides and mixtures thereof, which can be easily metabolized by beneficial strains of the skin or vaginal microflora, but which are scarcely or not capable of being metabolized by pathogenic or opportunistic strains.

**4.** Use according to claim 1, characterized in that the composition contains 0.1 to 20% by weight of said oligosaccharide.

**5.** Use according to claim 1, 2 or 3, characterized in that the oligosaccharide is a gluco-oligosaccharide of the general formula:

$$(O\text{-}\alpha\text{-D-glucopyranosyl})_n\text{-A}$$

where A is a residue of a glucose-accepting sugar chosen from maltose, isomaltose, isomaltotriose, methyl $\alpha$-glucoside and glucose, and $\underline{n}$ = 1 to 10, the glucoside bonds being of the $\alpha(1\rightarrow6)$ and, optionally, $\alpha(1\rightarrow2)$ and/or $\alpha(1\rightarrow3)$ type, the $\alpha(1\rightarrow2)$ bond, if present, being situated at the non-reducing end or constituting a branch point.

6. Use according to claim 1, 2 or 3, characterized in that the oligosaccharide is a fructo-oligosaccharide of one of the general formulae

GFn or Fm

where G is a glucose residue, F a fructose residue, n = 1 to 10 and m = 1 to 10.

7. Use according to claim 1, 2 or 3, characterized in that the oligosaccharide comprises an $\alpha$-galacto-oligosaccharide or a mixture of $\alpha$-galacto-oligosaccharides corresponding to the general formula:

$$(\alpha\text{-Gal}(1 \rightarrow 6))_n\, \alpha\text{-Glu}(1 \rightarrow 2)\beta\text{-fructose},$$

where n = 1, 2 or 3.

8. Use according to claim 1, 2 or 3, characterized in that the oligosaccharide comprises a mixture of $\beta$-galacto-oligosaccharides of general formula

$$(\beta\text{-Gal}\,(1 \rightarrow a))_n\, \beta\text{-Gal}\,(1 \rightarrow 4)\ \text{glucose}$$

where n = 1, 2 or 3 and a = 2, 3, 4 or 6, the $\beta(1 \rightarrow 6)$ isomer being predominant.

9. Use according to claim 1, characterized in that the composition comprises, in addition, a non-ionic surface-active agent.

10. Use according to claim 1, characterized in that the composition comprises, in addition, a buffer agent which adjusts the pH to the 5 to 7 range.

11. Use according to any one of claims 1 to 10, characterized in that the composition is in the form of a liquid soap, a shampoo, a body milk or a face cream.

12. Use according to any one of claims 1, 2 and 4 to 10, characterized in that the composition is in the form of a vaginal gel.

**Patentansprüche**

1. Verwendung einer Verbindung, ausgewählt aus der aus den Glucooligosacchariden, den Fructooligosacchariden, den $\alpha$- und $\beta$-Galactooligosacchariden und deren Mischungen bestehenden Gruppe, die durch günstige Stämme der Haut- oder Vaginal-Mikroflora leicht metabolisierbar sind, durch pathogene oder opportunistische Stämme jedoch wenig oder gar nicht metabolisierbar sind, für die Herstellung einer Zusammensetzung zur Behandlung der Haut oder der Vaginalschleimhaut zur Förderung der Entwicklung einer günstigen Haut- oder Vaginal-Mikroflora.

2. Verwendung einer Verbindung, ausgewählt aus der aus den Glucooligosacchariden, den Fructooligosacchariden, den $\alpha$- und $\beta$-Galactooligosacchariden und deren Mischungen bestehenden Gruppe, die durch günstige Stämme der Haut- oder Vaginal-Mikroflora leicht metabolisierbar sind, durch pathogene oder opportunistische Stämme jedoch wenig oder gar nicht metabolisierbar sind, für die Herstellung eines Mittels, das das Wachstum der günstigen Haut- oder Vaginal-Mikroflora fördert.

3. Kosmetische Verwendung eines das Wachstum der günstigen Haut-Mikroflora fördernden Produkts, umfassend eine Verbindung, ausgewählt aus der aus den Glucooligosacchariden, den Fructooligosacchariden, den $\alpha$- und $\beta$-Galactooligosacchariden und deren Mischungen bestehenden Gruppe, die durch günstige Stämme der Haut- oder Vaginal-Mikroflora leicht metabolisierbar sind, durch pathogene oder opportunistische Stämme jedoch wenig oder gar nicht metabolisierbar sind.

4. Verwendung nach Anspruch 1, dadurch gekennzeichnet, daß die Zusammensetzung 0,1 bis 20 Gew.-% des Oligosaccharids enthält.

5. Verwendung nach Anspruch 1, 2 oder 3, dadurch gekennzeichnet, daß es sich bei dem Oligosaccharid um ein Glucooligosaccharid der allgemeinen Formel:

$$(\text{O-}\alpha\text{-D-Glucopyranosyl})_n\text{-A}$$

handelt, wobei A ein Glucoseakzeptor-Zuckerrest, ausgewählt aus Maltose, Isomaltose, Isomaltotriose, Methyl-$\alpha$-Glucosid und Glucose, und $\underline{n}$ = 1 bis 10 ist, wobei die Glycosidbindungen vom Typ $\alpha(1\rightarrow6)$ und wahlweise $\alpha(1\rightarrow2)$ und/oder $\alpha(1\rightarrow3)$ sind, die Bindung $\alpha(1\rightarrow2)$, sofern vorhanden, sich am nichtreduzierenden Ende befindet oder eine Verzweigungsstelle darstellt.

6. Verwendung nach Anspruch 1, 2 oder 3, dadurch gekennzeichnet, daß es sich bei dem Oligosaccharid um ein Fructooligosaccharid mit einer der allgemeinen Formeln

$$\text{GFn oder Fm}$$

handelt, wobei G ein Glucoserest, F ein Fructoserest, n = 1 bis 10 und m = 1 bis 10 ist.

7. Verwendung nach Anspruch 1, 2 oder 3, dadurch gekennzeichnet, daß es sich bei dem Oligosaccharid um $\alpha$-Galactooligosaccharid oder eine Mischung von $\alpha$-Galactooligosacchariden handelt, die der allgemeinen Formel:

$$(\alpha\text{-Gal}(1\rightarrow6))_n,\alpha\text{-Glu}(1\rightarrow2)\text{-}\beta\text{-Fructose}$$

entsprechen, wobei n = 1, 2 oder 3.

8. Verwendung nach Anspruch 1, 2 oder 3, dadurch gekennzeichnet, daß das Oligosaccharid eine Mischung aus $\beta$-Galactooligosacchariden der allgemeinen Formel:

$$(\beta\text{-Gal}(1\rightarrow a))_n,\beta\text{-Gal}(1\rightarrow4)\text{-Glucose}$$

umfaßt, wobei n = 1, 2 oder 3 und a = 2, 3, 4 oder 6, wobei das Isomer $\beta(1\rightarrow6)$ überwiegt.

9. Verwendung nach Anspruch 1, dadurch gekennzeichnet, daß die Zusammensetzung darüber hinaus ein nichtionisches Tensid umfaßt.

10. Verwendung nach Anspruch 1, dadurch gekennzeichnet, daß die Zusammensetzung darüber hinaus einen Puffer umfaßt, wodurch der pH-Wert im Bereich von 5 bis 7 eingestellt wird.

11. Verwendung nach einem der Ansprüche 1 bis 10, dadurch gekennzeichnet, daß die Zusammensetzung in Form einer flüssigen Seife, eines Shampoos, einer Körpermilch oder einer Gesichtscreme vorliegt.

12. Verwendung nach einem der Ansprüche 1, 2 und 4 bis 10, dadurch gekennzeichnet, daß die Zusammensetzung in Form eines Vaginalgels vorliegt.